**Europäisches Patentamt**

**European Patent Office**

**Office européen des brevets**

(19)

(11) Veröffentlichungsnummer: **0 035 063**
**A2**

# EUROPÄISCHE PATENTANMELDUNG

(12)

(21) Anmeldenummer: **80107772.8**

(22) Anmeldetag: **10.12.80**

(51) Int. Cl.³: **G 02 B 1/10,** G 02 B 5/22,
A 61 N 5/06

(30) Priorität: **05.03.80 DE 3008364**

(43) Veröffentlichungstag der Anmeldung: **09.09.81**
**Patentblatt 81/36**

(84) Benannte Vertragsstaaten: **DE FR GB IT NL**

(71) Anmelder: **Röhm GmbH, Kirschenallee,**
**D-6100 Darmstadt (DE)**

(72) Erfinder: **Hosch, Ludwig, Grüner Weg 34,**
**D-6100 Darmstadt (DE)**
Erfinder: **Ittmann, Günther, Waldstrasse 11,**
**D-6114 Gross-Umstadt (DE)**

(54) **Eingefärbte Acrylglasabdeckungen für UV-Quellen.**

(57) Die Erfindung betrifft ein Mittel zur Abdeckung von
UV-emittierenden Strahlungsquellen auf der Basis von
Acrylglas, wobei das Acrylglas einen Gehalt an Farbmitteln
innerhalb eines Konzentrationsbereichs von 0,0005–0,3
Gew.-% (berechnet auf eine Schichtdicke der elektromagnetische Strahlung absorbierenden Matrix von 1 mm),
die im sichtbaren Bereich des Spektrums zwischen 400
und 550 nm absorbieren und bei einer in diesem Bereich
liegenden Wellenlänge eine Absorption von mindestens
2% besitzen und die gleichzeitig im A- und im B-Bereich
der UV-Strahlung einen spektralen Transmissionsgrad
$T_{(\lambda)}$ von im Mittel nicht unter 55% und bei keiner Wellenlänge in diesem Bereich von weniger als 10% besitzen.

Eingefärbte Acrylglasabdeckungen für
UV-Quellen

Als Abdeckungen von Lichtquellen, die UV-Licht
emittieren (UV-Strahlern), eignen sich Acrylgläser.

Voraussetzung für die Eignung als Abdeckungen
von UV-Strahlern ist einerseits möglichst weitgehende Durchlässigkeit für UV-Licht des gewünschten Wellenlängenbereichs, andererseits eine
hinreichende Unempfindlichkeit des Abdeckungsmaterials gegenüber der UV-Strahlung.
Eine besondere Bedeutung haben UV-Strahler auf
dem Sektor der Gesundheitsfürsorge erlangt, wo
UV-Strahler als "Künstliche Sonnen" oder "Höhensonnen" und in jüngerer Zeit als Solarien eine
weite Verbreitung gefunden haben.

Als UV-Strahler werden z.B. Quecksilberhochdruckbrenner und - in zunehmendem Maße - Niederdruckquecksilberdampflampen verwendet. Durch Verwendung
geeigneter Filter können Bereiche des UV-Spektrums,
die in gesundheitlicher Hinsicht bedenklich sind,
aus dem zur Wirkung kommenden UV-Licht eliminiert
werden. Bei der Anwendung der UV-Bestrahlung auf dem Gebiet der Gesundheitsvorsorge bzw. Kosmetik zeichnet sich die Tendenz ab, die UV-Bestrahlung auf die gesundheitlich unbedenklichen Spektralbereiche zwischen 400 und 315 nm (UVA-Bereich) und gewisse Anteile des Bereichs zwischen 315 und 280 nm (UVB-Bereich) zu beschränken. Der "harte" UVC-Bereich zwischen 280 und 200 nm soll möglichst vollständig ausgeschlossen werden. Die Bemühungen gehen dahin, den UVB-Anteil des zur Wirkung kommenden UV-Lichts wie bei der Sonnenstrahlung in der Größenordnung von 0,04 % zu halten.

Neben der herkömmlichen UV-Bestrahlung durch bewegliche UV-Strahler, die mehr auf die partienweise Bestrahlung des Körpers abzielte, sind sogenannte Solarien-Bänke entwickelt worden, bei der der menschliche Körper während der Bestrahlung oberhalb der Strahlungsquelle auf deren Abdeckung als Liegefläche aufliegt. Nach Herstellerangaben hat man der Auswahl des günstigsten UV-Bereichs große Aufmerksamkeit gewidmet, in dem Bemühen, die günstigen Wirkungen der Sonnenbestrahlung (antirachitische Wirkung, Bräunung usw.) zu erzielen, ohne dafür irgendwelche gesundheitlichen Nachteile in Kauf nehmen zu müssen. Ein besonderer Vorteil der Verwendung von Acrylglas als Abdeckung von beispielsweise Solarienbänken liegt, abgesehen von seiner Durchlässigkeit für elektromagnetische Strahlung bis herab zu ca. 260 nm darin, daß dieses Material wegen seiner geringen Wärmeleitzahl als angenehm bei Berührung empfunden wird. Hinzu tritt die Bruchunempfindlichkeit und leichte Bearbeitbarkeit der Acrylgläser, die im Bedarfsfalle auch vernetzt sein können. Allerdings lassen die UV-Bestrahlungsvorrichtungen des Standes der Technik im allgemeinen in einer bestimmten Wirkung zu wünschen übrig. Das von den UV-Strahlern emittierte sichtbare Licht, wird aufgrund seiner spektralen Zusammensetzung vom menschlichen Auge als "unnatürlich blau" und als "unangenehm kalt" empfunden. Aus diesem Eindruck kann eine ausgesprochene oder unausgesprochene Abneigung gegen die Anwendung von UV-Licht in der Gesundheitsfürsorge bzw. Kosmetik resultieren.

Es wurde nun gefunden, daß man Mittel zur Abdeckung von UV-emittierenden Strahlungsquellen auf der Basis von Acrylglas erhält, die die genannten Nachteile vermeiden, wenn das Acrylglas Farbmittel entweder in homogener Verteilung oder in Form einer nachträglichen Oberflächeneinfärbung im Tauchbad, als Lackierung oder als Bestandteil einer Farbfolie aufweist, die im sichtbaren Bereich von 400 bis 550 nm des elektromagnetischen Spektrums absorbieren und bei einer in diesem Bereich liegenden Wellenlänge eine Absorption von mindestens 2 % besitzen und die gleichzeitig im UVA-Bereich und im UVB-Bereich in einem Konzentrationsbereich von 0,005 - 0,3 % Gew.-%, berechnet auf eine Schichtdicke der elektromagnetischen Strahlung absorbierenden Matrix von 1 mm, einen spektralen Transmissionsgrad $\tau_{(\lambda)}$ von im Mittel nicht unter 55 %, und bei keiner Wellenlänge innerhalb dieses Bereichs einen Transmissionsgrad von weniger als 10 % besitzen. Besonders bevorzugt ist ein Farbmittelgehalt von 0,003 bis 0,1 Gew.-%.

Vorzugsweise finden solche Farbmittel Anwendung,
die bei 300 nm einen Transmissionsgrad von mindestens 20 % und bei 320 nm einen Transmissionsgrad von mindestens 50 % besitzen. Der vorstehend
und im folgenden verwendete Begriff "Farbmittel",
soll erfindungsgemäß einmal im Acrylglas homogen
verteilbare Substanzen mit der gewünschten spektralen, selektiven Absorption, also sowohl lösliche
Farbstoffe als auch Pigmente, zum anderen auch
solche Farbstoffe und/oder Pigmente, die sich als
Bestandteile von Lacken oder Folien eignen oder die
durch ein Tauchverfahren in die Oberfläche des Acrylglases aufgenommen werden. Die Farbmittel, die die gewünschten Eigenschaften mitbringen, sind an sich bekannt. Für durchsichtige Einfärbungen von Acrylgläsern verwendet man allgemein organische, im Polymerisat echt oder kolloidal lösliche Farbstoffe,
die sich auf eine sehr kleine Auswahl beschränken,
z.B. Azo-, Anthrachinon-, sowie einige Metallkomplexfarbstoffe. Spezifisch eignen sich als lösliche Farbstoffe für Acrylat-Einfärbungen insbesondere
Thermoplast®-, Neozapon®- und fettlösliche Farbstoffe,
wie Sudan®-Farbstoffe (Hersteller: BASF), Macrolex®-
und Ceres®-Farbstoffe (Hersteller: Bayer), Waxoline®-
Farbstoffe (Hersteller: ICI), sowie Solvaperm®-Farbstoffe und Fettfarbstoffe (Hersteller: Hoechst) und Orasol®- bzw. Oracet®-Farbstoffe und Mikrolith®-Farbstoffe (Hersteller: CIBA-GEIGY).

Bevorzugt sind solche Farbmittel, deren wellenlängenabhängige Absorption so bemessen ist, daß das durch die Acrylglas-Abdeckung hindurchtretende Licht vom Auge als gelb bis orange empfunden wird. Als bevorzugt werden für diesen Zweck Farbmittel aus der Gruppe der Anthrachinonfarbstoffe und/oder der Monoazofarbstoffe und/oder der Chrom-Komplex-Farbstoffe ausgewählt. Besonders genannt seien die Farbstoffe Makrolexorange GG® (Bayer) auf Anthrachinonbasis/Colour Index 58 050, Neozaponrange RE® (BASF) auf Chrom-Komplex, ICI: Solvapermorange 54. Vorzugsweise werden dem erfindungsgemäß zu verwendenden Acrylglas Lichtschutzmittel und/oder Stabilisatoren zugesetzt. Ihr Anteil beträgt in der Regel 0,01 bis 1 Gew.-%, bezogen auf die Monomeren. Bevorzugt sind sterisch gehinderte Amine, insbesondere Piperidinverbindungen, wie Bis-(2,2,6,6-tetramethyl-4-piperidyl)sebacat.

Die für Abdeckungen der beanspruchten Art brauchbaren Acrylgläser sind an sich bekannt, ebenso die dafür geeigneten Formverfahren. (Vgl. R. Vieweg et al. Kunststoff-Handbuch, Band IX, "Polymethacrylate", Carl Hanser Verlag 1975). Besonders genannt seien Acrylgläser auf der Basis von Methylmethacrylat. Als Comonomere kommen beispielsweise weitere Ester der Methacrylsäure bzw. Ester der Acrylsäure, insbesondere

von $C_1$ - $C_3$-Alkoholen infrage. Gleichfalls geeignet sind Vinylester von Alkansäuren, z.B. Vinylacetat. Limitierend für die Eignung ist in jedem Fall die Durchlässigkeit im UV-Bereich. Die Polymerisation kann zweckmäßigerweise nach dem Flachkammerverfahren (Kunststoff-Handbuch, Band IX, loc.cit.) durchgeführt werden. Bei der Durchfärbung der Matrix erfolgt Einrühren des Farbstoffs und des Lichtschutzmittels bzw. Stabilisators, gegebenenfalls eines Vernetzers und der erforderlichen Polymerisationsinitiatoren, wie Azo-Beschleuniger und/oder Perester, die selbst nicht im UV absorbieren dürfen. Der Gehalt an Initiator richtet sich innerhalb gewisser Grenzen nach der Stärke der Acrylglasplatte, er liegt im allgemeinen zwischen 0,01 und 0,2 Gew.-%, bezogen auf die Monomeren. Im allgemeinen wird die Polymerisation im Wasserbad zwischen 30 - 60°C eingeleitet und im Wärmeofen zwischen 90 und 120°C zu Ende geführt. Als Vernetzer kommen die an sich bekannten, mindestens zwei der Polymerreaktion zugängliche Doppelbindungen besitzenden Monomeren infrage. Genannt seien Glykoldimethacrylat oder Allylverbindungen, wie Triallylcyanurat oder Allylmethacrylat. Der Anteil an Vernetzern kann zwischen 0,01 und 10 Gew.-%, bezogen auf die Gesamtheit der Monomeren, betragen.

Als bekannt können ferner Verfahren zur Einfärbung von Acrylgläsern vorausgesetzt werden (vgl. Kunststoff-Handbuch, Band IX, loc.cit.). Die Oberflächenauftragung der Farbmittel kann ebenfalls in an sich bekannter Weise erfolgen. Die Lackierung kann gleichfalls in an sich bekannter Weise, z.B. durch Streichen, Siebdrucken oder Tauchen etc. vorgenommen werden.

Beispiele 1 - 7: Durchfärbung der Matrix

Grundansatz:

120 g Acrylsäuremethylester
4 g Bis-(2,2,6,6-tetramethyl-
4-piperidyl)-sebacat
1 g Azoisobuttersäuredinitril
875 g Methacrylsäuremethylester

Die Polymerisation des Grundansatzes, unter Zusatz der bei den einzelnen Beispielen genannten Farbmittel und Hilfsstoffe, wird im Flachkammerverfahren zwischen Silikatglasplatten distanziert, auf 6 mm Stärke zwischen 40 und 60°C innerhalb ca. 20 Stunden im Wasserbad durchgeführt und in einem Zeitraum von 6 - 10 Stunden zwischen 90 und 120°C wird endpolymerisiert.

Man erhält gelb bis orangefarbene, klar durchsichtige Platten, die gegebenenfalls nach Zuschnitt auf die erforderlichen Abmessungen, beispielsweise als Abdeckungen von Solarienliegen oder Lampen, oder auf dem Bausektor, z.B. als Bedachungen von Sonnenterrassen, verwendet werden können.

Beispiel (1)  Im oben genannten Grundansatz werden 0,0083 g Neozaponorange RE® gelöst und in einer Schichtdicke von 6 mm polymerisiert.

Beispiel (2)   Im Beispiel (1) genannten Ansatz werden 0,0166 g Neozaponorange RE$^®$ gelöst und in einer Schichtdicke von 6 mm polymerisiert.

Beispiel (3)   Im Beispiel (1) genannten Ansatz werden 0,0083 g Macrolexorange GG$^®$ gelöst und in einer Schichtdicke von 6 mm polymerisiert.

Beispiel (4)   Im Beispiel (1) genannten Ansatz werden 0,0166 g Macrolexorange GG$^®$ gelöst und in einer Schichtdicke von 6 mm polymerisiert.

Beispiel (5)   Zum Beispiel (1) genannten Ansatz werden 2 g Triallylcyanurat zugesetzt und 0,0083 g Neozaponorange RE$^®$ gelöst und in einer Schichtdicke von 6 mm polymerisiert.

Beispiel (6)   Zum Beispiel (1) genannten Ansatz werden 2 g Triallylcyanurat zugesetzt und 0,0083 g Macrolexorange GG$^®$ gelöst und in einer Schichtdicke von 6 mm polymerisiert.

Beispiel (7)   Zum Beispiel (1) genannten Ansatz werden 1 g Glycoldimethacrylat zugesetzt und 0,0083 g Macrolexorange GG$^®$ gelöst und in einer Schichtdicke von 6 mm polymerisiert.

In der folgenden Tabelle werden die Ergebnisse der Wärmeformbeständigkeit nach Vicat-Erweichungstemperatur (VET) sowie die Lichtdurchlässigkeit in % bei 300 nm ($\tau_{300}$) vor und nach Bestrahlung in verschiedenen Zeitintervallen (72, 250 und 1000 Stunden) mit einer Solarienlichtquelle (Lampentyp UVA=Solarium A1,00, 80W, 8Z (Wolf-System), UVB=Helarium B1,01, 80W, 8U (Wolf-System) angegeben.

T A B E L L E

| Beispiel | VET °C | \multicolumn{4}{c}{Lichtdurchlässigkeit in % bei 300 nm nach Bestrahlung} | \multicolumn{2}{c}{Bestrahlung mit UV} |
|---|---|---|---|---|---|---|---|
|  |  | Anl. | 72 h | 250 h | 1000 h | \multicolumn{2}{c}{Lampenart} |
|  |  |  |  |  |  | A | B |
| (1) | 103 | 59 | 57 | 59 | 62 | A |  |
|  |  | 60 | 58 | 59 | 62 |  | B |
| (2) | 103 | 50 | 48 | 50 | 53 | A |  |
|  |  | 50 | 47 | 49 | 52 |  | B |
| (3) | 101 | 62 | 57 | 60 | 63 | A |  |
|  |  | 62 | 60 | 63 | 64 |  | B |
| (4) | 103 | 58 | 52 | 54 | 55 | A |  |
|  |  | 58 | 54 | 55 | 55 |  | B |
| (5) | 103 | 58 | 54 | 57 | 58 | A |  |
|  |  | 58 | 54 | 54 | 55 |  | B |
| (6) | 102 | 63 | 57 | 59 | 60 | A |  |
|  |  | 62 | 58 | 59 | 59 |  | B |
| (7) | 102 | 65 | 58 | 61 | 64 | A |  |
|  |  | 65 | 59 | 62 | 64 |  | B |

Beispiel 8: a) Oberflächeneinfärbung

Das einzufärbende Material auf der Basis des farblosen Acrylglases des Grundansatzes der vorstehenden Beispiele wird je nach der gewünschten Farbstärke in die entsprechenden Farblösungen eingetaucht.

Lösung I    0,5 g Oracetgelb 5GN® vorgelöst in
300,0 g Aceton wird mit
200,0 g dest. $H_2O$ versetzt.

Lösung II    0,25 g Oracetorange 2R® vorgelöst in
300,0 g Aceton wird mit
200,0 g dest. $H_2O$ versetzt.

| Lösung | Tauchzeit sec | Temperatur der Lösung | Aussehen des resultierenden Acrylglases |
|--------|---------------|------------------------|------------------------------------------|
| I | 5 | 40°C | sehr helles leuchtendes gelb |
| I | 20 | 40°C | hellgelb klar |
| II | 5 | 40°C | sehr helles orange |
| II | 10 | 40°C | orange klar |

b) Lackierung

Farblack: 0,1 g Makrolexorange GG® wird in 100 g eines UV-durchlässigen Acrylharzlacks oder eines Nitrolacks gelöst.

Auf eine farblose Acrylglasplatte auf der Basis des unter Beispiel 1 beschriebenen Grundansatzes wird eine geeignete Menge des Farblacks nach Maßgabe der gewünschten Farbnuancierung aufgetragen. Die Auftragung erfolgt durch Bestreichen. Alternativ kann auch getaucht, gespritzt oder siebgedruckt werden.

Mit vergleichbarem Resultat können analog den Beispielen 1 - 8 auch Färbungen mit Zaponechtorange RE® vorgenommen werden.

Eingefärbte Acrylglasabdeckungen für
UV-Quellen

_____

Patentansprüche

1. Mittel zur Abdeckung von UV-emittierenden
Strahlungsquellen auf der Basis von Acrylglas, gekennzeichnet durch einen Gehalt an
Farbmitteln innerhalb eines Konzentrationsbereichs von 0,005 - 0,3 Gew.-% (berechnet
auf eine Schichtdicke der elektromagnetische
Strahlung absorbierenden Matrix von 1 mm),
die im sichtbaren Bereich des Spektrums
zwischen 400 und 550 nm absorbieren und bei
einer in diesem Bereich liegenden Wellenlänge
eine Absorption von mindestens 2 % besitzen
und die gleichzeitig im A- und im B-Bereich
der UV-Strahlung einen spektralen Transmissionsgrad $\tau_{(\lambda)}$ von im Mittel nicht unter
55 % und bei keiner Wellenlänge in diesem
Bereich von weniger als 10 % besitzen.

2. Mittel gemäß Anspruch 1, dadurch gekennzeichnet,
daß die Farbmittel bei 300 nm einen spektralen
Transmissionsgrad $\tau_{300}$ von mindestens 20 % und
bei 320 nm einen Transmissionsgrad $\tau_{320}$ von
mindestens 50 % besitzen.

3. Mittel gemäß den Ansprüchen 1 und 2, dadurch gekennzeichnet, daß die Farbmittel in das Acrylglas eingebracht und/oder nachträglich als Oberflächeneinfärbung im Tauchbad, als Lackierung oder als Bestandteile einer Folie auf das Acrylglas aufgebracht werden.

4. Mittel gemäß den Ansprüchen 2 und 3, dadurch gekennzeichnet, daß die Farbmittel, eine solche Absorption besitzen, daß das durch die Abdeckung durchtretende Licht vom Auge als gelb bis rot empfunden wird.

5. Mittel gemäß Anspruch 4, dadurch gekennzeichnet, daß die Farbmittel aus der Gruppe der Anthrachinonfarbstoffe und/oder der Azofarbstoffe und/oder der Chrom-Komplexreihe gewählt werden.

6. Mittel gemäß den Ansprüchen 1 bis 5, dadurch gekennzeichnet, daß der Gehalt an Farbmitteln 0,0005 bis 0,3 Gew.-%, vorzugsweise 0,003 bis 0,1 Gew.-%, bezogen auf eine Materialdicke von 1 mm, beträgt.

7. Mittel gemäß den Ansprüchen 1 bis 6, dadurch gekennzeichnet, daß neben dem Farbmittel noch Stabilisatoren und/oder Lichtschutzmittel und wahlweise Vernetzungsmittel verwendet werden.

8. Mittel gemäß Anspruch 7, dadurch gekennzeichnet, daß als Stabilisatoren sterisch gehinderte Amine verwendet werden.

9. Mittel gemäß den Patentansprüchen 7 und 8, dadurch gekennzeichnet, daß als Stabilisatoren Piperidinverbindungen, vorzugsweise Bis-(2,2,6,6-Tetramethyl-4-piperidyl)sebacat, verwendet werden.

10. Mittel gemäß den Patentansprüchen 1 bis 9, dadurch gekennzeichnet, daß als Acrylglas Polymethylmethacrylat, das gegebenenfalls weitere Copolymere enthält, verwendet wird.

11. Verwendung der geformten Mittel gemäß den Ansprüchen 1 bis 10, zur Abdeckung von UV-emittierenden Strahlungsquellen.